# EUROPÄISCHE PATENTANMELDUNG

(11) **EP 4 371 467 A1**
(43) Veröffentlichungstag der Anmeldung: **22.05.2024**
(21) Anmeldenummer: 22075014.5
(22) Anmeldetag: 18.11.2022
(51) Int. Cl.: A61B 1/267, A61B 1/32

(54) **INTUBATIONSGERÄT**

(71) Anmelder: Raymondos, Konstantinos, 30655 Hannover (DE)
(72) Erfinder: Raymondos, Konstantinos, 30655 Hannover (DE)
(74) Vertreter: Günther, Constantin

(57) **Zusammenfassung**

Die Erfindung betrifft ein Intubationsgerät zum Intubieren eines Patienten mit einem Endotrachealtubus, mit folgenden Merkmalen:
a) einem Handgriff zum Halten des Intubationsgeräts,
b) eine mit dem Handgriff verbundene starre Führungsschiene, die einen in Längsrichtung von einem patientenfernen Ende bis zu einem patientennahen Ende der Führungsschiene verlaufenden Führungskanal zum Führen des Endotrachealtubus und eine beim Intubationsvorgang der Zunge des Patienten zugewandte Oberseite hat,
c) einem Epiglottisheber zum Anheben der Epiglottis des Patienten, wobei der Epiglottisheber Ober wenigstens ein Lagerungselement an einem Bauteil des Intubationsgeräts beweglich gelagert ist,
d) wenigstens eine optische Erfassungseinrichtung im patientennahen Bereich an der Führungsschiene und/oder wenigstens eine optische Erfassungseinrichtung im patientennahen Bereich an dem Epiglottisheber,
e) wobei der Epiglottisheber zumindest im patientennahen Bereich, insbesondere in einem Biegebereich der Führungsschiene, die Führungsschiene oder zumindest den Führungskanal auf der Oberseite der Führungsschiene überwiegend oder vollständig geschlossen überdeckt.

## Beschreibung

Die Erfindung betrifft ein Intubationsgerät zum Intubieren eines Patienten mit einem Endotrachealtubus. Allgemein gesagt betrifft die Erfindung das Gebiet der Laryngoskopie.

### Stand der Technik zur Laryngoskopie und unterschiedlichen Techniken zur Eröffnung der oberen Atemwege

Prinzipiell lassen sich vier Laryngoskopietechniken bzw. Eröffnungstechniken der oberen Atemwege unterscheiden:
1. Endoskopische Laryngoskopie mit flexiblem oder starrem Endoskop.
2. Standardlaryngoskopie mit einem Spatel oder Mundspreizer.
3. Ausschließliche Eröffnung des tiefen Rachenbereiches und Anheben der Epiglottis.
4. Eröffnung der Atemwege mit einem sogenannten "oropharyngealen Atemweg"

Gemeinsam ist allen Techniken, dass der tiefe Rachenbereich vor dem Larynx eröffnet und die Epiglottis so angehoben ist, dass der Larynxeingang frei liegt. Bei der endoskopischen Laryngoskopie geschieht das spontan durch den Patienten. Bei den anderen Techniken wird der tiefe Rachenbereich mit einem Hilfsgerät aktiv so eröffnet, dass auch die Epiglottis angehoben wird. Die beiden unter 2. und 3. genannten Methoden unterscheiden sich darin, dass bei der Standardlaryngoskopie auch Druck auf die oberen Rachenanteile, die Halsweichteile und den Zungenboden ausgeübt und der Mund zusätzlich eröffnet werden muss.

Bei der endoskopischen Laryngoskopie wird ein flexibles oder starres Endoskop so eingeführt, dass der Larynxeingang betrachtet werden kann. Da das in den allermeisten Fällen beim wachen oder sedierten Patienten erfolgt, sind die Atemwege im tiefen Rachenbereich meistens eröffnet Eine Eröffnung der Atemwege mit einem Hilfsgerät ist daher meistens nicht erforderlich.

Die Endoskop-Optiken werden hierbei weder durch einen Spatel noch andere Weise geschützt und es gibt auch keine andere Vorrichtung, die die Atemwegsschleimhaut oder Atemwegssekrete auf sicheren Abstand zu der Optik halten können. Bei zu tiefer Sedierung, nach Narkoseeinleitung oder im Koma fallen die Atemwege zusammen und die Atemwegsschleimhaut und/oder Sekret blockieren die Sicht. In der klinischen Routine wird dann versucht, die Optik frei zu spülen.

Um eine flexible Optik um die Zunge und die Epiglottis herum zu führen, werden auch sogenannte "oropharyngeale Atemwege" eingesetzt. Allerdings ist bei Patienten in Narkose oder im Koma die Öffnung und insbesondere das Anheben der Epiglottis oft unzureichend, um sich sicher und schnell orientieren zu können.

Beim Einführen gerader, starrer Endoskope wird auch versucht, die oberen Atemwege mit dem Daumen offen zu halten. In der Regel würde man dann aber ein Standardlaryngoskop verwenden, um die Zunge nach oben zu verdrängen und dann auch indirekt die Epiglottis anzuheben. Auch kann das starre Endoskop über einen Mundspreizer oder ein sogenanntes "starres Rohr" eingeführt werden:
Das starre Rohr stellt die einfachste Form eines Endoskops dar: Hiermit wird bei überstrecktem Kopf eines üblicherweise narkotisierten Patienten ein Rohr so eingeführt, dass der Kehlkopfeingang auch ohne Optik direkt wie bei der Standardlaryngoskopie zu sehen ist. Dementsprechend müssen wie bei der Standardlaryngoskopie ebenfalls auch die oberen Rachenanteile, die Halsweichteile und der Zungenboden verdrängt werden.

Bei der Standardlaryngoskopie wird nicht nur der tiefe Rachenbereich eröffnet und die Epiglottis angehoben: Um die Öffnung in der Tiefe zu erreichen und den hierfür nötigen Spateldruck effektiv auf das Gewebe im tiefen Rachenbereich übertragen zu können, muss der Druck auch auf den oberen Rachenbereich und auf die Zunge ausgeübt werden. Bei der konventionelien, direkten Laryngoskopie wird somit auch Druck auf den oberen Rachenbereich, auf die Zunge und über die Halsweichteile auch auf den Unterkiefer ausgeübt und der Mund muss weiter eröffnet werden. So wird versucht, den Kehlkopfeingang direkt einzusehen. Dabei kann es erforderlich sein, den Kopf zu überstrecken.

In der HNO erfolgt nach dem gleichen Prinzip wie die Standardlaryngoskopie eine Laryngoskopie mit Hilfe eines Mundspreizers. Hierbei wird in Narkose bei überstrecktem Kopf der Spatel zum Offenhalten der Atemwege fixiert und über einen am Oberkiefer fixierten Bogen auch der Mund in verschiedenen Stufen offen gehalten (z.B. Mundöffner nach Davis-Boyle).

Bei der ausschließlichen Eröffnung des tiefen Rachenbereiches und Anheben der Epiglottis wird ausschließlich der tiefe Rachenbereich eröffnet, so dass die Epiglottis angehoben und der Larynxeingang freigelegt wird. Im Gegensatz zu den o.g. Standardverfahren wird kein bzw. sehr viel weniger Druck auf die oberen Rachenanteile und die Zunge ausgeübt und der Mund muss nicht zusätzlich eröffnet werden. Ein Beispiel ist in der EP 1 439 776 B1 angegeben. Das dort beschriebene Intubationsgerät wird in den Mund eingeführt und im Rachen ohne Sichtkontrolle in der Tiefe positioniert. Dann wird die Führungsschiene nach vorn bzw. nach unten geschoben. Der die Schiene umschließende Bügel bleibt im Griff fixiert. Durch die so erfolgende Trennung von Führungsschiene und Bügel wird der tiefe Rachenraum eröffnet. Durch die Öffnung des tiefen Rachenraumes wird bei korrekter Lage des Geräts auch die Epiglottis angehoben und somit der Kehlkopfeingang freigelegt. Dann kann eine Intubation erfolgen - allerdings ohne führende Sichtkontrolle.

Eröffnung der Atemwege mit einem sogenannten "oropharyngealen Atemweg": 1908 wurde erstmals ein oropharyngealer Atemweg beschrieben und ein weiches Modell aus Gummi wurde 1933 von Güdel eingeführt. Der oropharyngeale Standardatemweg wird immer noch als "Güdeitubus" bezeichnet. Bei bewusstlosen oder sedierten Patienten wird ein oropharyngealer Atemweg eingeführt, um die oberen Atemwege bei noch erhaltener Spontanatmung offen zu halten. Auch wird er nach Narkoseeinleitung eingeführt, um die Maskenbeatmung zu erleichtern. An einem J-förmigen Rohr soll ein Flansch am oberen Ende ein Verschlucken verhindern. Güdeltuben liegen in vielen Größen vor und sie werden im Notfall häufig und auch ohne ärztliche Anordnung von medizinischem Personal angewendet.

Es gibt sehr viele Variationen, wobei einige davon für eine Intubation ohne Sicht entwickelt wurden. Später wurden sie für eine endoskopische Untersuchung der Atemwege und auch für eine Intubation mit Sicht mithilfe eines Endoskops benutzt.

### Probleme und Nachteile bei der endoskopischen Laryngoskopie

Eine endoskopischen Laryngoskopie mit starren oder flexiblen Optiken beim ungesicherten Atemweg kann in der Regel nur erfolgen, wenn die Atemwege durch den wachen oder nicht zu tief sedierten Patienten selbst offen gehalten werden. Hierbei ist das Sichtfeld bei den Standardendoskopen sehr viel kleiner im Vergleich zur Standardiaryngoskopie, was die Orientierung insbesondere für Anfänger sehr erschweren kann.

Bei zu tiefer Sedierung, in Narkose oder bei Koma kollabieren die oberen Atemwege. Eine Orientierung zwischen den aneinanderheftenden Schleimhäuten kann dann sehr erschwert oder sogar unmöglich werden. Darüber hinaus ist eine Kontamination der freiliegenden und damit ungeschützten Optik zwischen den Schleimhäuten mit Sekret sehr viel wahrscheinlicher, so dass die Optik freigespült werden muss. Bei nicht effektiver Spülung ist die Sicht allerdings blockiert und eine Unterscheidung zwischen Kontamination durch Sekret und anliegender Schleimhaut wird unmöglich. Liegt in den kollabierten Atemwegen die Schleimhaut direkt auf der ungeschützten Optik kann auch bei effektiver Spülung keine Sicht und somit keine Orientierung erzielt werden. Bei kollabierten Atemwegen kann mit verschiedenen, sogenannten Atemwegsmanövern versucht werden, die oberen Atemwege zu öffnen. Hierzu gehören das Anheben des Unterkiefers und/oder das Überstrecken des Kopfes. Auch kann versucht werden, mit dem Daumen die Zunge nach oben zu drücken.

### Probleme und Nachteile bei den weiteren Techniken

Um den tiefen Rachenbereich öffnen und die Epiglottis anheben zu können, muss - wie oben beschrieben- bei der Standardiaryngoskopie zusätzlich Druck auch auf den oberen Rachenbereich und auch auf fast die gesamte Zunge ausgeübt werden. Hierbei muss in der Regel auch der Mund zusätzlich weiter eröffnet und der Unterkiefer durch Druck über die Zunge und die Halsweichteile angehoben und dann in dieser Position während der Intubation auch mit der Hand gehalten werden.

Um diesen hierbei erforderlichen, sehr hohen Druck auf den oberen Rachenbereich, auf die Zunge und über die Halsweichteile auch auf den Unterkiefer auszuüben, ist mitunter sehr viel Kraft erforderlich. Die Kraft muss dann noch erhöht werden, wenn bei einer schwierigen Laryngoskopie beispielsweise der Kopf nicht überstreckt und/oder z.B. bei pathologischen Prozessen verhärtete Halsweichteile oder anders anatomisch veränderte obere Atemwegsstrukturen nicht ausreichend angehoben und verdrängt werden können. Dann kann es sehr schwierig oder sogar unmöglich sein, eine direkte Sicht bei der konventionellen Laryngoskopie oder mit dem starrem Rohr zu erlangen oder die Optik eines Videolaryngoskops oder Endoskops in die gewünschte Position zu bringen. Daher ist eine Atemwegssicherung mittels Intubation sehr erschwert oder unmöglich gemacht.

Sogar bei der normalen, problemlosen Standardlaryngoskopie handelt es sich um einen sehr invasiven Vorgang. Zahnschäden gehören daher auch zu den häufigsten Komplikationen der Allgemeinanästhesie. Wegen der großen Krafteinwirkung werden immer sehr ausgeprägte Abwehrreflexe mit entsprechenden Stressreaktionen ausgelöst. Das hat weitreichende Konsequenzen für jede Narkoseeinleitung bei der auf diese Weise intubiert wird und bei der versucht werden muss, diese Stressreaktionen durch Narkosemittel zu vermeiden. Diese Narkosemittel haben allerdings mitunter erhebliche Nebenwirkungen, die bei älteren Patienten und insbesondere bei Patienten mit bestimmten Vorerkrankungen lebensbedrohlich sein können.

Mitunter kann sogar eine Intubation deswegen erschwert sein, weil die Intubierenden selbst bei normalen Atemwegen einfach nicht in der Lage sind, die erforderliche Kraft aufzubringen. Um bei HNO Eingriffen am Larynx diese Kraft nicht aufbringen zu müssen und zusätzlich noch eine Hand frei zu haben, wird dann ein Mundspreizer zur Laryngoskopie verwendet: Hierbei wird die erforderliche Kraft über Hebelwirkungen durch eine Stütze auf der Brust und einer zusätzlichen Fixierung am Oberkiefer aufgebracht. Wegen der durch diese Krafteinwirkung entstehenden Belastung müssen mit Mundspreizer durchgeführte Eingriffe immer in Narkose durchgeführt werden.

Der Erfindung liegt die Aufgabe zugrunde, ein universell anwendbares Intubationsgerät anzugeben, das ein zügiges, sicheres und patientenschonendes Intubieren auch unter Narkose erlaubt.

Diese Aufgabe wird gelöst durch ein Intubationsgerät zum Intubieren eines Patienten mit einem Endotrachealtubus, mit folgenden Merkmalen:
a) einem Handgriff zum Halten des Intubationsgeräts, insbesondere zum Halten mit einer ganzen Hand,
b) eine mit dem Handgriff verbundene Führungsschiene, die einen in Längsrichtung von einem patientenfernen Ende bis zu einem patientennahen Ende der Führungsschiene verlaufenden Führungskanal zum Führen des Endotrachealtubus und eine beim Intubationsvorgang der Zunge des Patienten zugewandte Oberseite hat,
c) einem Epiglottisheber zum Anheben der Epiglottis des Patienten, wobei der Epiglottisheber über wenigstens ein Lagerungselement an einem Bauteil des Intubationsgeräts beweglich gelagert ist,
d) wenigstens eine optische Erfassungseinrichtung im patientennahen Bereich an der Führungsschiene und/oder wenigstens eine optische Erfassungseinrichtung im patientennahen Bereich an dem Epiglottisheber,
e) wobei der Epiglottisheber zumindest im patientennahen Bereich, insbesondere in einem Biegeabschnitt der Führungsschiene, die Führungsschiene oder zumindest den Führungskanal auf der Oberseite der Führungsschiene überwiegend oder vollständig geschlossen überdeckt.

Der Epiglottisheber ist somit in dem Bereich, in dem er die Führungsschiene oder zumindest den Führungskanal auf der Oberseite der Führungsschiene überdeckt, überwiegend oder vollständig geschlossen ausgebildet und bildet hierdurch ein Dach über der wenigstens einen optischen Erfassungseinrichtung im patientennahen Bereich an der Führungsschiene und/oder der wenigstens einen optischen Erfassungseinrichtung im patientennahen Bereich an dem Epiglottisheber. Auf diese Weise ist zumindest die Erfassungsseite der optischen Erfassungseinrichtung, z.B. eine Linse, eine Objektiv oder eine Kamera, unterhalb des überwiegend oder vollständig geschlossenen Bereichs des Epiglottishebers angeordnet (sozusagen unter dem Dach) und dadurch vor einer Sichtblockade durch Zungengewebe oder durch Verschmutzung geschützt.

Die Merkmale des Intubationsgeräts sind dabei so zu verstehen, dass das Intubationsgeräts durch seine Führungsschiene für das Führen des Endotrachealtubus geeignet ist. Selbstverständlich ist es möglich, auch andere Tubus-ähnliche Gegenstände mit dem Intubationsgerät in einen Patienten einzuführen, wie z.B. ein Endoskop. Die Führungsschiene kann vorteilhaft starr ausgebildet sein, sie kann somit im bestimmungsgemäßen Betrieb des Intubationsgeräts zumindest im wesentlichen nicht verformt werden.

Die optische Erfassungseinrichtung kann insbesondere als Kamera ausgebildet sein. Die optische Erfassungseinrichtung erlaubt bei in den Patienten eingeführtem Intubationsgerät einen ungestörten Blick in den Kehlkopfeingang auf die Stimmbänder.

im unbetätigten Zustand des Epiglottishebers ist der Epiglottisheber zumindest im patientennahen Bereich auf der Oberseite der Führungsschiene angeordnet, z.B. indem er dort auf der Führungsschiene aufliegt. Vorteilhafterweise kann der Epiglottisheber an einem patientenfernen Ende ein Bedienelement zum manuellen Betätigen durch einen Anwender haben.

Allgemein kann gesagt werden, dass im unbetätigten Zustand des Epiglottishebers sich der patientennahe Bereich des Epiglottishebers näher an der Führungsschiene befindet als im betätigten Zustand. Wird der Epiglottishebers manuell betätigt, zum Beispiel durch Ziehen am Bedienelement, wird hierdurch der patientennahe Bereich des Epiglottishebers vom benachbarten Bereich der Führungsschiene fortgezogen.

Das Bedienelement kann insbesondere für die Aufnahme einer Zugkraft beim Betätigen des Epiglottishebers eingerichtet sein, insbesondere eine vom patientennahen Ende fortweisende Zugkraft. Der Anwender muss somit am Bedienelement eine Zugkraft aufbringen, um den Epiglottisheber zu betätigen.

Der Handgriff kann an der Unterseite der Führungsschiene angeordnet sein. Der Handgriff kann bezüglich der Unterseite der Führungsschiene im rechten Winkel abragen oder leicht in Richtung des patientennahen Endes geneigt und damit schräg zur Unterseite der Führungsschiene angeordnet sein, zum Beispiel in einem Winkel zwischen 60 und 90 Grad.

Gemäß einer vorteilhaften Ausgestaltung der Erfindung ist vorgesehen, dass der Epiglottisheber am patientenfernen Ende des überwiegend oder vollständig geschlossenen Bereichs in einen offenen Bereich übergeht, der eine schlitzförmige Aussparung hat, die seitlich (links und rechts der schlitzförmigen Aussparung) jeweils von einem Seitensteg begrenzt ist. Anders gesagt, zwischen den Seitenstegen ist ein innerer offener Längsschlitz ausgebildet. Durch den inneren offenen Längsschlitz ist der Führungskanal nicht überdeckt und somit frei zugänglich. Die Seitenstege können in das Bedienelement münden. Auf diese Weise wird die Führungsschiene vom Epiglottisheber nicht vollständig überdeckt, sondern ist insbesondere in einem patientenfernen Bereich durch den Längsschlitz des Epiglottishebers frei zugänglich. Beispielsweise kann der Endotrachealtubus dann in diesem Bereich durch den Längsschlitz in den Führungskanal der Führungsschiene eingelegt und mit der Hand so am patientennahen Teil des Endotrachealtubus gefasst und vorgeschoben werden, dass der Endotrachealtubus an den Stimmbändern vorbei sicher in die Luftröhre geführt werden kann.

Gemäß einer vorteilhaften Ausgestaltung der Erfindung ist vorgesehen, dass der Epiglottisheber in dem überwiegend oder vollständig geschlossenen Bereich entgegengesetzt U-förmig zur Führungsschiene ausgebildet ist. Hierdurch wird eine gute Stabilität und hohe Steifigkeit des Epiglottishebers erreicht. Zudem ist die Einheit aus der Führungsschiene und dem Epiglottisheber sehr flach bauend, so dass sie gut auch in enge Atemwege eingeführt werden kann. Auf diese Weise kann ein universelles intubationsgerät geschaffen werden, das für alle Größen und Altersgruppen von Patienten geeignet ist, das heißt das sich für alle Größen von Atemwegen eignet (One size fits all).

Gemäß einer vorteilhaften Ausgestaltung der Erfindung ist vorgesehen, dass der Epiglottisheber zumindest in dem überwiegend oder vollständig geschlossenen Bereich die Führungsschiene beidseitig übergreift. Der Epiglottisheber ist somit zumindest in diesem Bereich breiter als die Führungsschiene. Dies erlaubt eine mechanisch einfache und zuverlässige Gleitlagerung des Epiglottishebers auf der Führungsschiene.

Gemäß einer vorteilhaften Ausgestaltung der Erfindung ist vorgesehen, dass der Epiglottisheber über das wenigstens eine Lagerungselement relativ zum Handgriff bewegbar ist, so dass durch eine Relativbewegung des Epiglottishebers gegenüber dem Handgriff die Epiglottis beim Intubationsvorgang anhebbar ist. Dies erlaubt eine ergonomisch günstige Betätigung. Die gesamte Einheit aus der Führungsschiene und dem Epiglottisheber kann vom Anwender mit einer Hand am Handgriff gehalten werden. Zum Betätigen des Epiglottishebers muss der Handgriff nicht in seiner Lage verändert werden, es ist lediglich eine manuelle Betätigung des Epiglottishebers am Bedienelement durch die andere Hand des Anwenders erforderlich.

Gemäß einer vorteilhaften Ausgestaltung der Erfindung ist vorgesehen, dass der Handgriff am patientenfernen Ende der Führungsschiene starr mit der Führungsschiene gekoppelt ist. Zumindest beim Betätigen des Epiglottishebers erfolgt keine Relativbewegung der Führungsschiene gegenüber dem Handgriff.

Gemäß einer vorteilhaften Ausgestaltung der Erfindung ist vorgesehen, dass der Epiglottisheber über das wenigstens eine Lagerungselement an der Führungsschiene gleitend verschiebbar und/oder schwenkbar gelagert ist. Auch hierdurch wird eine einfache und ergonomische manuelle Bedienung des Intubationsgeräts gefördert. Beispielsweise kann der Epiglottisheber entlang eines geradlinig verlaufenden oder geringfügig gekrümmten Bereichs der Führungsschiene verschiebbar sein.

Gemäß einer vorteilhaften Ausgestaltung der Erfindung ist vorgesehen, dass der Epiglottisheber sich im Wesentlichen über die gesamte Längserstreckung der Führungsschiene erstreckt. Der Epiglottisheber kann auch am patientennahen Ende und/oder am patientenfernen Ende über die Führungsschiene hinaus ragen.

Gemäß einer vorteilhaften Ausgestaltung der Erfindung ist vorgesehen, dass der Epiglottisheber der Formgebung der Führungsschiene in Längsrichtung folgt. Dementsprechend kann der Epiglottisheber über seine gesamte Längserstreckung eng an der Führungsschiene anliegen, so dass das gesamte Intubationsgerät zumindest ist in den Abschnitten, die in einen Patienten einzuführen sind, besonders flach bauend ist.

Gemäß einer vorteilhaften Ausgestaltung der Erfindung ist vorgesehen, dass die Einheit aus der Führungsschiene und dem Epiglottisheber zumindest im unbetätigten Zustand des Epiglottishebers über die gesamte Längserstreckung eine Bauhöhe hat, die weniger als 50 % größer als die Bauhöhe der Führungsschiene an der jeweiligen Stelle ist.

Gemäß einer vorteilhaften Ausgestaltung der Erfindung ist vorgesehen, dass die Bauhöhe der Einheit aus der Führungsschiene und dem Epiglottisheber Ober die gesamte Längserstreckung geringer ist als die Breite dieser Einheit, insbesondere maximal 75 % der Breite.

Gemäß einer vorteilhaften Ausgestaltung der Erfindung ist vorgesehen, dass der Epiglottisheber auf der vom Handgriff abgewandten Seite der Führungsschiene angeordnet und/oder gelagert ist, insbesondere in allen beim Intubieren wählbaren Verstellpositionen des Epiglottishebers. Dies erlaubt eine ergonomisch günstige Benutzung des Intubationsgeräts.

Gemäß einer vorteilhaften Ausgestaltung der Erfindung ist vorgesehen, dass der Epiglottisheber stufenlos oder feinstufig in einer Vielzahl von Stufen relativ zum Handgriff bewegbar ist. Dies hat den Vorteil, dass der Epiglottisheber je nach Größe der Atemwege des Patienten an einer beliebigen Stelle positioniert werden kann, soweit es für das Öffnen tiefen Rachenbereiches und das Anheben der Epiglottis erforderlich ist. Es muss somit nicht eine konstruktiv vorgegebene Mindestverstellung des Epiglottishebers durchgeführt werden. Hierdurch kann das Intubationsgerät besonders universell und schonend bei Patienten mit sehr unterschiedlichen Atemwegen eingesetzt werden. Insbesondere kann der Epiglottisheber stufenlos oder feinstufig relativ zum Handgriff vor und wieder zurück bewegt werden, also in beiden Verstellrichtungen, so dass die passende Verstellposition durch Probieren leicht gefunden werden kann. Eine feinstufige Verstellbarkeit kann z.B. wenigstens 10 oder wenigstens 20 Stufen umfassen. Der Epiglottisheber kann z.B. rastend an der Führungsschiene entlag bewegbar sein.

Gemäß einer vorteilhaften Ausgestaltung der Erfindung ist vorgesehen, dass das Intubationsgerät eine erste Fixiervorrichtung hat, mit der eine eingestellte Relativposition des Epiglottishebers relativ zum Handgriff stufenlos oder feinstufig in einer Vielzahl von Stufen fixierbar ist. Grundsätzlich ist es möglich, dass eine eingestellte Relativposition des Epiglottishebers vom Anwender mit einen Finger, zum Beispiel einen Finger der Hand, die den Handgriff hält, fixiert wird. Durch ein zusätzliches mechanisches Fixierelement, wie zum Beispiel die erste Fixiervorrichtung, kann eine noch sicherere und dauerhafte Fixierung erfolgen. Zudem kann die Handhabung für den Anwender vereinfacht werden, da der Anwender die Fixierung nicht dauerhaft manuell aufrechterhalten muss. Beispielsweise kann die erste Fixiervorrichtung ähnlich wie eine Klammer oder ein Clip ausgebildet sein, die Ober die Führungsschiene und den Epiglottisheber geklemmt wird.

Gemäß einer vorteilhaften Ausgestaltung der Erfindung ist vorgesehen, dass der Epiglottisheber lösbar an einem Bauteil des Intubationsgeräts befestigt ist, so dass der Epiglottisheber nach Durchführung des Intubationsvorgangs separat von der Führungsschiene vom Patienten entfernbar ist. Dies hat den Vorteil, das nach erfolgter Intubation die einzelnen Teile des Intubationsgeräts separat voneinander vom Patienten entfernt werden können. Es kann zunächst der Epiglottisheber entfernt werden, dann die Baueinheit mit der Führungsschiene.

Gemäß einer vorteilhaften Ausgestaltung der Erfindung ist vorgesehen, dass der Epiglottisheber wenigstens ein erstes formschlüssiges Befestigungselement hat, das zumindest im unbetätigten Zustand des Epiglottishebers im formschlüssigen Eingriff mit einem zweiten formschlüssigen Befestigungselement des Intubationsgeräts ist. Dementsprechend ist der Epiglottisheber nicht nur lose auf der Führungsschiene aufgelegt, sondern zusätzlich durch das erste formschlüssige Befestigungselement daran befestigt. Hierdurch wird verhindert, dass sich der Epiglottisheber vor dem Einfühen des Intubationgerätes in den Patienten unerwünscht lösen und herunterfallen kann.

Dabei sind das erste und zweite formschlüssige Befestigungselement zusätzlich zum wenigstens einen Lagerungselement vorhanden. Vorteilhafter Weise sind erstes und zweites formschlüssiges Befestigungselement nur im unbetätigten Zustand des Epiglottishebers im formschlüssigen Eingriff miteinander, d.h. nach geringfügiger Betätigung des Epiglottishebers lösen sie sich voneinander, z.B. bei einem Betätigungsweg von weniger als 3 cm. Dies ist wiederum förderlich für ein einfaches Separieren des Epiglottishebers von der Führungsschiene nach erfolgter Intubation.

Gemäß einer vorteilhaften Ausgestaltung der Erfindung ist vorgesehen, dass die Führungsschiene zumindest in einem patientennahen Bereich einen Biegeabschnitt aufweist, in dem die Führungsschiene von der Seite aus betrachtet gebogen ist, z.B. vom Handgriff aus gesehen konvex gebogen. Der Biegeabschnitt kann z.B. einen 90 Grad-Bogen umfassen, oder einen Bogen von etwas mehr oder weniger als 90 Grad, z.B. etwa 80 Grad. Vorteilhaft ist z.B. ein Biegeabschnitt, der sich über einen Bogen von 70 bis 90 Grad erstreckt. Dies ist förderlich für eine zielgerichtete, zügige Einführung der Führungsschiene in die Atemwege eines Patienten, so dass der Patient bestmöglich geschont wird.

Gemäß einer vorteilhaften Ausgestaltung der Erfindung ist vorgesehen, dass der Führungskanal auf der Innenseite des Biegeabschnitts überwiegend oder vollständig offen ist. Auch dies ist förderlich für eine flachbauende Konstruktion des Intubationsgerätes. Der Führungskanal kann auf der Oberseite auch außerhalb des des Biegeabschnitts überwiegend oder vollständig offen sein, auch über die gesamte Länge. Insbesondere kann die Führungsschiene zumindest im Biegeabschnitt oder insgesamt ein U-förmiges Profil haben.

Gemäß einer vorteilhaften Ausgestaltung der Erfindung ist vorgesehen, dass die Führungsschiene am patientenfernen Ende des Biegeabschnitts in einen linear verlaufenden oder im Vergleich zum Biegeabschnitt weniger stark gekrümmten Abschnitt übergeht. Dies ermöglicht insbesondere eine gleitende Linearführung oder quasi-lineare Führung des Epiglottishebers auf der Führungsschiene. Dabei kann die Führungsschiene im weniger stark gekrümmten Abschnitt einen wesentlich größeren Biegeradius haben als im Biegeabschnitt.

Gemäß einer vorteilhaften Ausgestaltung der Erfindung ist vorgesehen, dass das Intubationsgerät eine zweite Fixiervorrichtung hat, mit der die Lage des Intubationsgeräts relativ zum Patienten, insbesondere die Winkellage, stufenlos oder feinstufig in einer Vielzahl von Stufen fixierbar ist. Durch die zweite Fixiervorrichtung kann insbesondere der Kippwinkel des Intubationsgerätes relativ zum Patienten festgelegt werden. Beispielsweise kann die zweite Fixiervorrichtung ein Kugelgelenk oder Kardangelenk haben, das in einer gewünschten Winkellage fixiert werden kann, zum Beispiel mittels einer Klemmvorrichtung. Die zweite Fixiervorrichtung kann zum Beispiel auf dem Kiefer des Patienten abgestützt sein.

Auf diese Weise kann das in den Patienten eingeführte und in einer gewünschten Position fixierte Intubationsgerät auch die Funktion eines oralen Atemweges übernehmen. Mit dem Intubationsgerät ist auf diese Weise der Atemweg gesichert, wobei vorteilhafterweise durch die optische Erfassungseinrichtung eine Sicht in den Atemweg hinein möglich ist und dadurch die korrekte Lage sicher bestätigt werden kann.

Das erfindungsgemäße Intubationsgerät vereint einen optimalen Blickwinkel auf die Stimmlippen mit optimaler Führbarkeit eines Tubus über den Kehlkopfeingang in die Luftröhre. Es eignet sich hervorragend zur einfacheren und sichereren Atemwegssicherung mittels Intubation für den schwierigen und normalen Aternweg. Das erfindungsgemäße Intubationsgerät erlaubt ein effektives Öffnen der Atemwege, eine zuverlässige Laryngoskopie und eine sichere Intubation in verschiedenen klinischen Situationen. Damit bietet das erfindungsgemäße Intubationsgerät eine Vielzahl von Vorteilen:
1. Nur der tiefe Rachenbereich wird so eröffnet, dass die Epiglottis effektiv angehoben und eine freie Sicht auf den gesamten Larynxeingang ermöglicht wird. Der Querdurchmesser im Mund- und oberen Rachenbereich wird hierbei nicht weiter vergrößert und daher erfolgt hier auch keine oder nur sehr geringe Krafteinwirkung, Im Unterschied zu bekannten Geräten ist es z.B. nicht erforderlich, beim oder vor dem Öffnen des tiefen Rachenbereichs und Anheben der Epiglottis auch die oberen Bereiche der Atemwege in eine Linie zu bringen und insbesondere den Mund mit zu eröffnen und zu weiten. Bei der konventionellen Laryngoskopie, aber auch bei der Videolaryngoskopie ist hierfür mitunter ein erheblicher Druck mit entsprechend sehr hoher Krafteinwirkung erforderlich, weswegen hierbei in der Regel eine Vollnarkose durchgeführt wird, was beim erfindungsgemäßen Intubationsgerät nicht unbedingt erforderlich ist.
2. Durch einen sehr geringen Abstand zwischen dem patientennahen Ende des Epiglottishebers und der Erfassungsseite der optischen Erfassungseinrichtung kann die Erfassungsseite direkt vor den Larynxeingang positioniert werden, um eine vollständige Sicht auf die Glottis zu erhalten. Darüber hinaus kann das patientennahe Ende des Epiglottishebers um die Zunge herum geführt werden, anstatt sie zu verdrängen.
3. Durch einen geringen Querdurchmesser (Bauhöhe) der Einheit aus der Führungsschiene und dem darauf angeordneten Epiglottisheber unter 2 cm wird das Einführen in den Mund erleichtert.
4. Eine in der Führungsschiene und/oder dem Epiglottisheber integrierte optische Erfassungseinrichtung wird durch den Epiglottisheber in den meisten klinischen Routinefällen effektiv vor einer Sichtblockade durch vorgelagertes Zungengewebe oder durch eine Verschmutzung mit Sekret oder Zungenbelag geschützt.
5. Das Öffnen des tiefen Rachenbereichs und das Anheben der Epiglottis erfolgt stufenlos und bedarfsgerecht unter Sichtkontrolle.
6. Der Epiglottisheber kann nach oben und wieder nach unten bewegt und somit die Position unter Sichtkontrolle korrigiert und nach Bedarf angepasst werden.
7. Führungsschiene und Epiglottisheber können sehr einfach und schnell voneinander getrennt und nach Intubation problemlos aus dem Mund entfernt werden. Dadurch wird die Gefahr einer akzidentellen Extubation beim Entfernen des Geräts direkt nach der Intubation minimiert.
8. Die entscheidende optische Erfassungseinrichtung für eine effektive Laryngoskopie mit vollständiger Sicht auf die Glottis vor, während und nach der Intubation befindet sich an der Führungsschiene. Diese Sicht verändert sich bei Eröffnung des tiefen Rachenbereichs nicht, da Epiglottisheber und Führungsschiene unabhängig voneinander bewegt werden: Das bedeutet, dass trotz Epiglottisheberbewegungen zum öffnen des tiefen Rachenbereiches und Anheben der Epiglottis die Glottissicht sich entsprechend der konstanten Position der Führungsschiene sich ebenfalls nicht verändert.
   Die Ergänzung einer optischen Erfassungseinrichtung am Epiglottisheber stellt eine Zusatzoption für eine unterstützende Übersicht dar, die besonders bei veränderter Anatomie und großen Flüssigkeitsansammlungen von Bedeutung ist.
9. Ohne die Bewegungsfreiheit oder die anderen oben genannten Eigenschaften des Epiglottishebers einzuschränken, kann die erfolgte Öffnungs- oder Schließbewegung (s.o. 5. und 6.) unter Sichtkontrolle an der gewünschten Stelle auf einfache Weise fixiert werden. Das kann beispielsweise mit einem Befestigungsclip erreicht werden. Durch die optischer Kontrolle erfolgt eine genaue individuelle Anpassung, wodurch ein sehr effektiver, oropharyngealer Atemweg geschaffen wird.
10. Durch die Fixierung in einer geöffneten Stellung wird beim liegenden Patienten durch das Eigengewicht von Zunge und Halsweichteilen die Führungsschiene des Intubationsgeräts an die Rachenhinterwand gedrückt und selbständig In der eingestellten Position gehalten. Ein Festhalten mit der Hand oder eine anderweitige Fixierung ist nicht erforderlich.

Wie man erkannt, ist es vorteilhaft, wenn der Epiglottisheber an die Führungsschiene angepasst ist. Der Epiglottisheber kann bei Betätigung an die Führungsschiene entlang gleiten und wird von dieser geführt. Die Führungsschiene hat somit eine doppelte Führungsfunktion, nämlich erstens zum Führen des Intubationstubus und zweitens zum Führen des Epiglottishebers. Durch diese Führung kann der Epiglottisheber nicht seitlich von der Führungsschiene herunterrutschen. Ansonsten kann der Epiglottisheber überwiegend lose auf der Führungsschiene aufgesetzt sein, z.B. indem der Epiglottisheber nur durch die Fixierung über die ersten und zweiten formschlüssigen Befestigungselemente im Bereich der unbetätigten Position an der Führungsschiene gehalten ist.

Hinsichtlich der gebogenen Formgebung im Biegeabschnitt der Führungsschiene kann der Epiglottisheber auch daran angepasst sein, z.B. indem er einen in Seitenansicht gebogenen Bereich hat, der sich über einen Bogenabschnitt von 70 bis 90 Grad erstreckt. In diesem gebogenen Bereich kann der Epiglottisheber überwiegend oder vollständig geschlossen ausgebildet sein und hiermit das erwähnte Dach über der Führungsschiene ausbilden. An den gebogenen Bereich kann sich ein in Seitenansicht gerade verlaufender Bereich des Epiglottishebers anschließen. Der überwiegend oder vollständig geschlossen ausgebildete Abschnitt des Epiglottishebers kann am Übergang zum geraden Bereich enden oder sich etwas, z.B. max 4. cm, in diesen geraden Bereich hinein erstrecken. Hieran schließt sich in Richtung zum patientenfernen Ende hin der mit den Seitenstegen und der zwischen den Seitenstegen ausgebildeten schlitzförmigen Aussparung gebildete Abschnitt an, der dann in das Bedienelement übergeht. Die Seitenstege können im Querschnitt als einfach abgewinkeltes Profil ausgebildet sein.

Im Sinne der vorliegenden Erfindung ist unter dem unbestimmten Begriff "ein" kein Zahlwort zu verstehen. Wenn also z.B. von einem Bauteil die Rede ist, so ist dies im Sinne von "mindestens einem Bauteil" zu interpretieren. Soweit Winkelangaben in Grad gemacht werden, beziehen sich diese auf ein Kreismaß von 360 Grad (360°).

Die Erfindung wird nachfolgend anhand von Ausführungsbeispielen unter Verwendung von Zeichnungen näher erläutert.

Es zeigen
- Figur 1: ein Intubationsgerät in rückwärtiger Ansicht,
- Figur 2: das Intubationsgerät in Seitenansicht im unbetätigten Zustand,
- Figur 3: das Intubationsgerät in Seitenansicht im betätigten Zustand,
- Figur 4: das Intubationsgerät in Seitenansicht in einem weiteren betätigen Zustand,
- Figur 5: den Epiglottisheber in einer Ansicht auf die Unterrseite,
- Figur 6: den Epiglottisheber in einer Ansicht auf die Oberseite,
- Figur 7: den Epiglottisheber In Seitenansicht,
- Figur 8: den Epiglottisheber in perspektivischer Ansicht auf die Oberseite,
- Figur 9: den Epiglottisheber in perspektivischer Ansicht auf die Unterseite,
- Figur 10: das intubationsgerät in Seitenansicht beim Lösen des Epiglottishebers,
- Figur 11: das Intubationsgerät ohne Epiglottisheber in perspektivischer Ansicht auf die Oberseite,
- Figur 12: das Intubationsgerät ohne Epiglottisheber in Draufsicht auf die Oberseite,
- Fig. 13 - 20: weitere Ausführungsformen von Intubationsgeräten und Epiglottishebern jeweils in Seitenansicht.

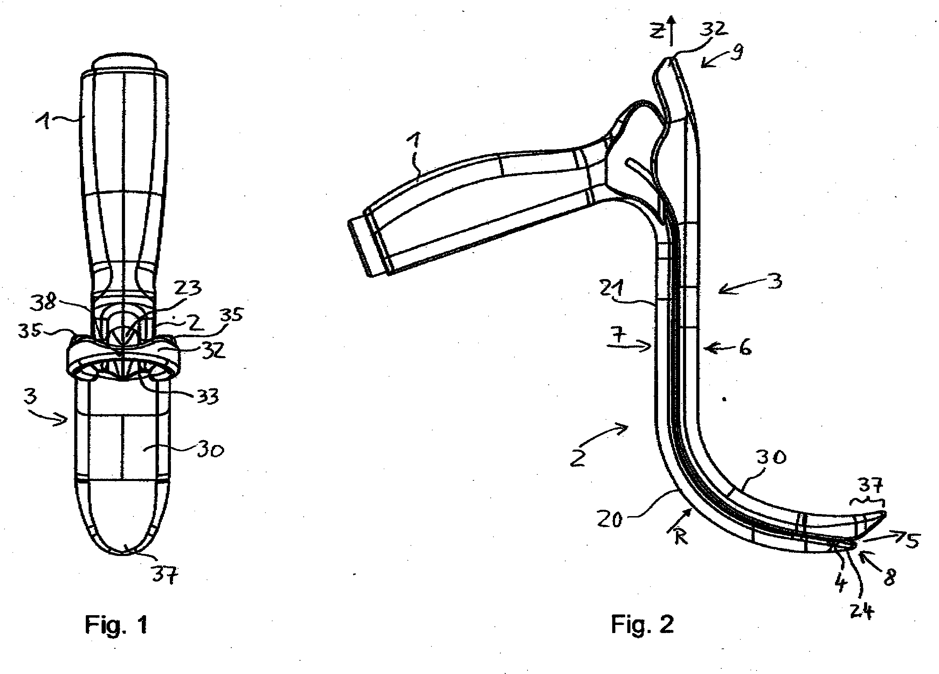

Das in den Figuren 1 und 2 dargestellte Intubationsgerät hat einen Handgriff 1 zum Halten des Intubationsgeräts. Mit dem Handgriff 1 kann das Intubationsgerät insbesondere während der Intubation geführt werden. Der Handgriff 1 ist dazu eingerichtet, dass er vom Anwender mit der ganzen Hand gegriffen werden kann. Das Intubationsgerät hat eine starre Führungsschiene 2, die ebenfalls starr mit dem Handgriff 1 verbunden ist. Der Handgriff 1 ragt von einer Unterseite 7 der Führungsschiene 2 ab und bildet zu der Unterseite 7 der Führungsschiene 2 einen Winkel von weniger als 90 Grad, zum Beispiel im Bereich von 70 Grad. Der Handgriff 1 ist auf diese Weise zum patientennahen Bereich 8 der Führungsschiene 2 hin etwas schräg gestellt.

Im patientennahen Bereich 8 endet die Führungsschiene 2 mit einem Biegeabschnitt 20, in dem die Führungsschiene 2 in Seitenansicht mit einem gewissen Radius R gekrümmt ausgebildet ist. Nahe des freien Endes 24 der Führungsschiene 2 ist eine optische Erfassungseinrichtung 4 in die Führungsschiene 2 integriert. Der Biegeabschnitt 20 kann sich über einen Winkelbereich von zum Beispiel 70 bis 100 Grad erstrecken, wobei ein Winkel von etwas unter 90 Grad besonders vorteilhaft ist. Vom patientennahen Bereich 8 aus gesehen schließt sich an den Biegeabschnitt 20 ein in Seitenansicht gerade verlaufender Bereich 21 der Führungsschiene 2 an. Dieser gerade verlaufende Bereich 21 mündet in den Handgriff 1. Die Führungsschiene 2 ist im Querschnitt U-förmig ausgebildet und dementsprechend zur Oberseite 6 hin offen. Ober die gesamte Längserstreckung der Führungsschiene 2 verläuft darin ein Führungskanal 23, in dem ein Endotrachealtubus entlang der Führungsschiene 2 geführt werden kann. Der Führungskanal 23 ist links und rechts von erhabenen Randseiten der Führungsschiene 2 begrenzt.

Auf der Oberseite 6 der Führungsschiene 2 ist ein Epiglottisheber 3 angeordnet. Wie man insbesondere in der Figur 2 erkennt, folgt der Epiglottisheber 3 im wesentlichen der Formgebung der Führungsschiene 2, das heißt der Epiglottisheber 3 ist im gerade verlaufenden Bereich 21 der Führungsschiene 2 ebenfalls im Wesentlichen gerade verlaufend ausgebildet Im Biegeabschnitt 20 der Führungsschiene 2 ist der Epiglottisheber 3 in analoger Weise gebogen ausgebildet. Erst am patientennahen freien Ende kann der Epiglottisheber 3 mit einer anderen Formgebung fortgeführt werden, insbesondere kann er über das freie Ende 24 der Führungsschiene 2 im patientennahen Bereich 8 etwas hinaus stehen.

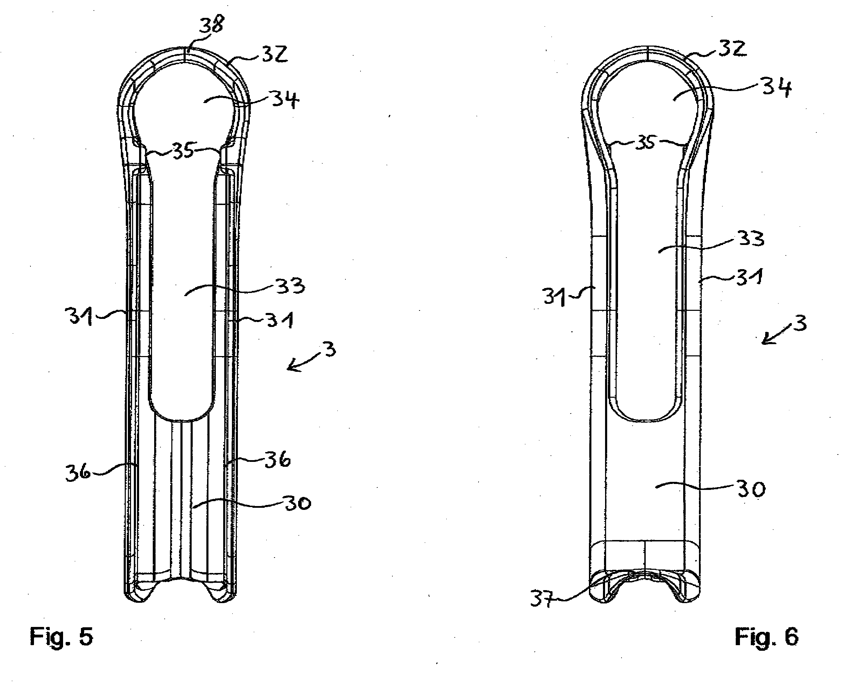

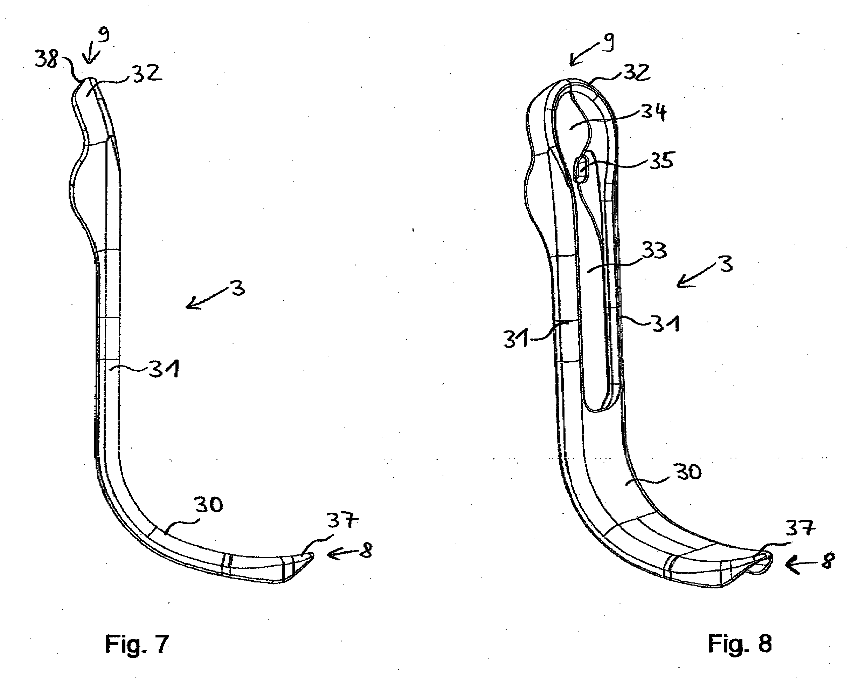

Wie insbesondere durch die Figuren 5 bis 9 verdeutlicht wird, ist der Epiglottisheber 3 im patientennahen Bereich 8 mit einem geschlossenen Abschnitt 30 gestaltet, so dass er eine Art Dach über der nach oben offenen Führungsschiene 2 bildet und in diesem Bereich entgegengesetzt U-förmig auf der Führungsschiene 2 angeordnet ist. In Richtung zum patientenfernen Bereich 9 schließt sich an den geschlossenen Abschnitt 30 des Epiglottisheber 3 ein teilweise offener Abschnitt an, der durch jeweils links und rechts an der Führungsschiene 2 angeordnete Seitenstege 31 gebildet wird. Zwischen den Seitenstegen 31 befindet sich eine schlitzförmige Aussparung 33, durch die der Führungskanal 23 zugänglich ist. Die Seitenstege 31 münden im patientenfernen Bereich 9 in ein Bedienelement 32 des Epiglottishebers 3, das wie erkennbar ist ringförmig ausgebildet ist.

Innerhalb des ringförmigen Bedienelements 32 befindet sich eine Bedienaussparung 34, in der das Bedienelement 32 vom Anwender z.B. mit dem Daumen und dem Zeigefinger gegriffen werden kann. Vorteilhafterweise ist die Abmessung (Durchmesser) des ringförmigen Bedienelements 32 beziehungsweise der durch die Bedienaussparung 34 geschaffene Freiraum etwas breiter als die Führungsschiene 2. Dies erlaubt das Hindurchführen des vollständigen Endotrachealtubus mit Konnektor durch die Bedienaussparung 34. Der Epiglottisheber 3 kann eine in Richtung zum Handgriff 1 gewandte Verjüngung 38 an der Mitte der Oberkante des Bedienelements 32 haben, die das Einführen des Endotrachealtubus oder eines Katheters auch von der dem Handgriff 1 zugewandten Seite des Epiglottishebers 3 direkt auf die Führungsschiene 2 ermöglicht, ohne durch die schlitzförmige Aussparung 33 des Epiglottishebers 3 hindurchzutreten.

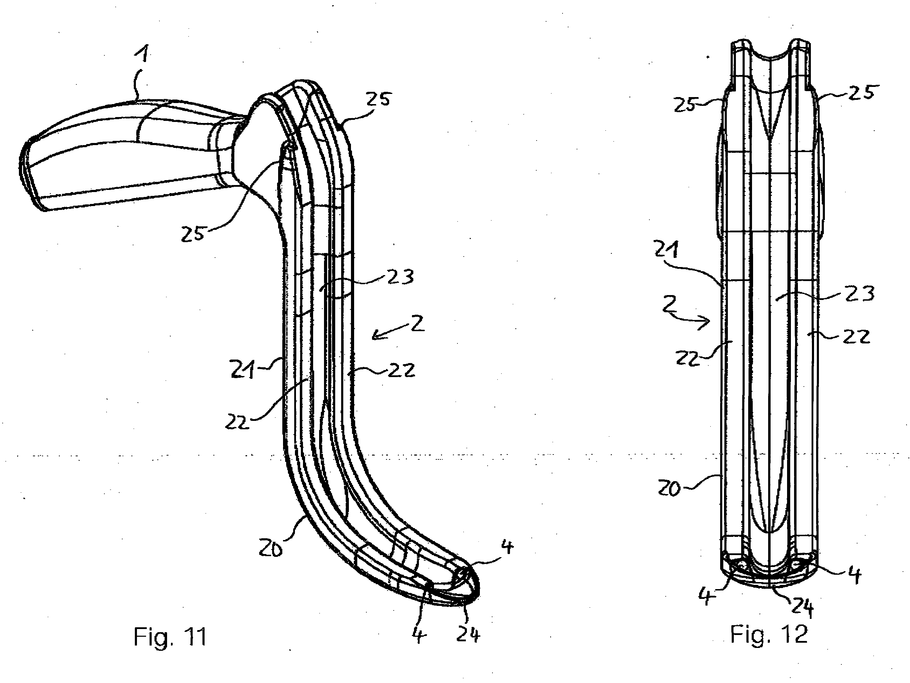

Die den Führungskanal 23 ist links und rechts begrenzenden erhabenen Randseiten der Führungsschiene 2 bilden mit Ihren zum Epiglottisheber 3 gewandten Oberflächen Auflagerungsflächen 22 zur Auflagerung des Epiglottishebers 3, wie in den Figuren 11 und 12 erkennbar ist. Der Epiglottisheber 3 ist mit seinen Seitenstegen 31 auf den Auflagerungsflächen 22 aufgelagert und kann darauf gleitend verschoben werden. Hierfür haben die Seitenstege 31 an den zur Führungsschiene 2 gewandten Seiten Auflagerungsflächen 36, die auf den Aufiagerungsflächen 22 aufliegen. Die Auflagerungsflächen 22 bilden damit Lagerungselemente für den Epiglottisheber 3. Der Epiglottisheber 3 kann etwas breiter als die Führungsschiene 2 sein und dementsprechend die Führungsschiene 2 außen übergreifen. Hierdurch wird eine zuverlässige schienenartige Längsführung des Epiglottisheber 3 auf der Führungsschiene 2 geschaffen.

Des Weiteren sind an dem Epiglottisheber 3 an den Innenseiten der Seitenstege 31 oder im Übergang zum Bedienelement 32 jeweils links und rechts erste formschlüssige Befestigungselemente 35 angeformt, zum Beispiel in Form von nach innen abragenden Zapfen oder Vorwölbungen. Des Weiteren befinden sich in diesem Bereich zweite formschlüssige Befestigungselemente 25 an der Führungsschiene 2, die zum Beispiel im Bereich des Übergangs der Führungsschiene 2 zum Handgriff 1 angeordnet sein können, zum Beispiel in Form von nach außen abragenden Zapfen oder Vorwölbungen. Die zweiten formschlüssigen Befestigungselemente 25 sind zumindest in der unbetätigten Stellung des Epiglottishebers 3, wie in den Figuren 1 bis 2 dargestellt, in formschlüssigem Eingriff mit den ersten formschlüssigen Befestigungselementen 35, um den Epiglottisheber 3 an der Führungsschiene 2 zu halten. Durch diese formschlüssige Fixierung kann der Epiglottisheber 3 insbesondere nicht in einer Richtung weg von der Oberseite 6 der Führungsschiene bewegt werden, das heißt in der Ansicht der Figur 2 nicht nach rechts bewegt werden.

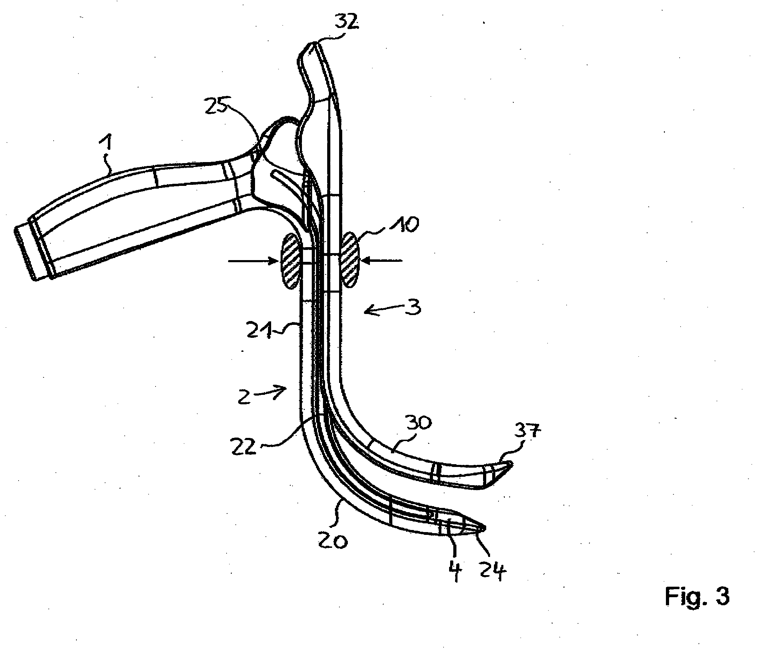

Wird der Epiglottisheber 3 durch Aufbringen einer Zugkraft Z am Bedienelement 32 betätigt, verschiebt er sich in der Ansicht der Figur 2 etwas nach oben, das heißt der gekrümmte patientennahe Bereich 30 des Epiglottishebers 3 entfernt sich dann vom Biegeabschnitt 20 der Führungsschiene 2. Im gerade verlaufenden Bereich gleitet dabei der Epiglottisheber 3 auf dem geraden Bereich 21 der Führungsschiene 2 entlang. Die Figur 3 zeigt das Intubationsgerät mit dem auf diese Weise betätigten Epiglottisheber 3. Ab einer gewissen Betätigungsposition sind dann die formschlüssigen Befestigungselemente 25, 35 nicht mehr im Angriff miteinander, so dass der Epiglottisheber 3 zum Beispiel mittels einer Schwenkbewegung von der Führungsschiene 2 weggeklappt werden kann, zum Beispiel nach Durchführung des Intubationsvorgangs, wenn der Epiglottisheber 3 vom Patienten entfernt werden soll.

Wie die Figur 3 ebenfalls zeigt, kann der Epiglottisheber 3 in der betätigten Position, in der der tiefe Rachenbereich eines Patienten eröffnet ist, mit einer ersten Fixiervorrichtung 10 an der Führungsschiene 2 fixiert werden, so dass die Position gehalten wird. Durch die erste Fixiervorrichtung 10 wird der Epiglottisheber 3 gegen die Führungsschiene 2 gedrückt, wie durch die Pfeile in Figur 3 verdeutlicht wird.

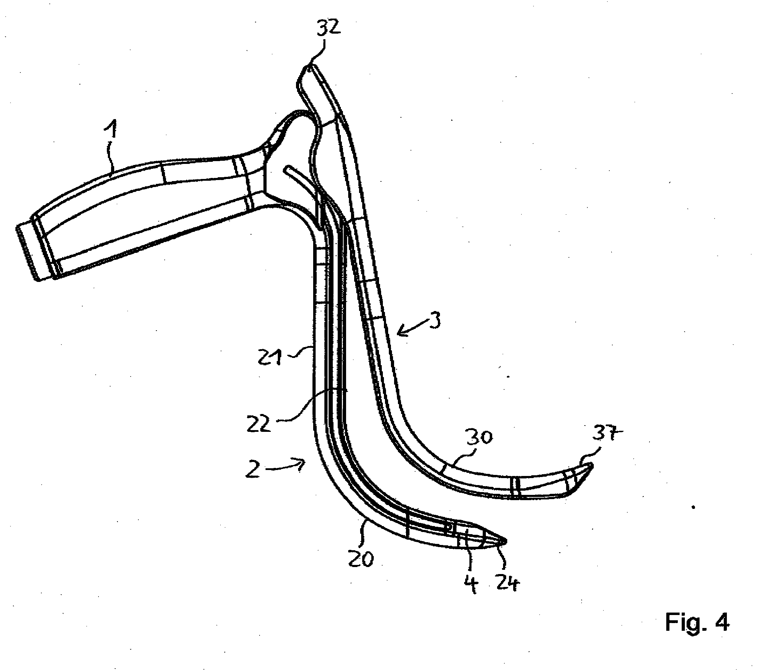

Die Figur 4 zeigt eine alternative Möglichkeit der Betätigung des Epiglottishebers 3 in Form eines Verschwenkens oder Kippens des Epiglottishebers 3 gegenüber der Führungsschiene 2. Hierfür ist der Epiglottisheber 3 schwenkbar an der Führungsschiene 2 gelagert. Durch Betätigen des Bedienelemnts 32 wird der patientennahe Teil des Epiglottishebers 3 von der Führungsschiene 2 fortgeschwenkt.

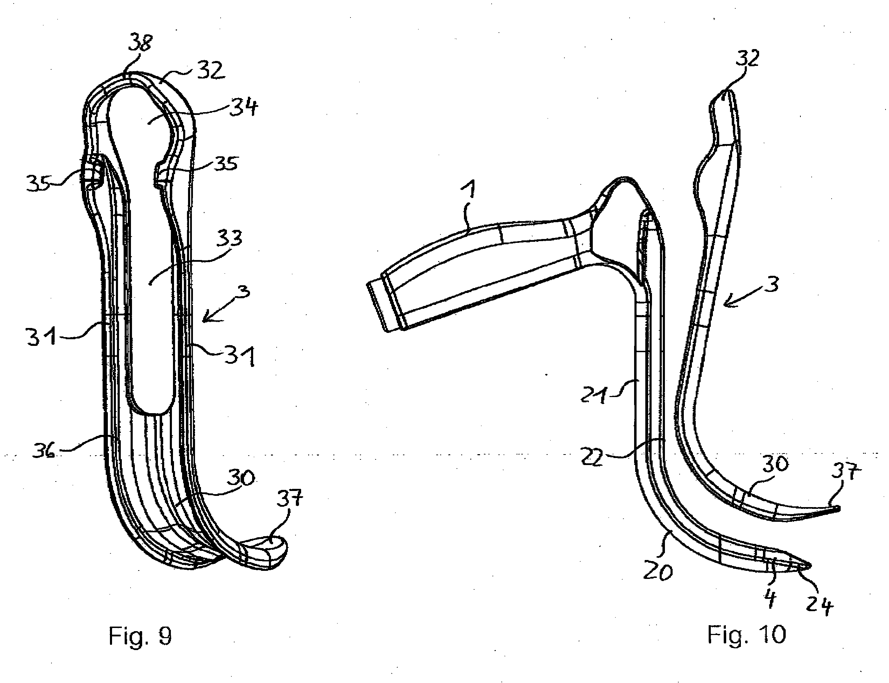

In der betätigten Position, wie z.B. in Figur 3 gezeigt, kann der Epiglottisheber 3 von der Führungsschiene 2 entfernt werden, wie die Figur 10 verdeutlicht.

### Eröffnung ausschließlich des tiefen Rachenbereiches

Der Epiglottisheber 3 ist genau auf die Führungsschiene 2 angepasst. Gleitet der Epiglottisheber 3 durch Betätigung am Bedienelement 32 mit der Zugkraft Z auf der Führungsschiene 2 nach oben, vergrößert sich der Abstand zwischen Führungsschiene 2 und Epiglottisheber 3 nur im unteren patientennahen Bereich 8, im oberen patientenfernen Bereich 9 bleibt die geringe Bauhöhe erhalten. Hierdurch wird der tiefe Rachenbereich eröffnet und durch Anheben der Epiglottis der Larynxeingang freigelegt.

Der Epiglottisheber 3 wird z.B. mit der linken Hand nach oben gezogen und mit der rechten Hand, die auch den Handgriff 1 hält, in der gewünschten Position gehalten: Hierfür ziehen Daumen und Zeigefinger der linken Hand am Bedienelement 32 mit der Zugkraft Z. Dadurch gleitet der Epiglottisheber 3 auf der Führungsschiene 2 entlang nach oben und der tiefe Rachenbereich wird eröffnet. Mit dem Zeigefinger der rechten Hand kann der jeweilige Abstand stufenlos, bedarfsgerecht und sicher gehalten werden. Der Zeigefinger der rechten Hand drückt hierfür den Epiglottisheber 3 gegen die Führungsschiene 2, während mit dem Daumen und anderen Fingern der rechten Hand der Handgriff 1 gehalten wird.

Diese beiden Einzelkomponenten werden aufeinander abgestimmt gleichzeitig angewandt: Der Epiglottisheber 3 gleitet beim Hochziehen unter der Spitze des Zeigefingers auf der Führungsschiene 2 nach oben. Wird der Epiglottisheber 3 mit der Zeigefingerspitze an die Führungsschiene 2 gedrückt, wird hierdurch die gewünschte Position fixiert und auch während der Intubation sicher gehalten.

Das Hochziehen mit der linken und Halten mit der rechten Hand erfolgen gleichzeitig. Unter optischer Kontrolle mittels der optischen Erfassungseinrichtung 4 kann so stufenlos und bedarfsgerecht die gewünschte Höhe eingestellt und auch während der Intubation problemlos gehalten werden.

### Länge und Krümmung der Spatelspitze 37

Als Spatelspitze 37 wird dabei das patientennahe (aus Anwendersicht distale) Ende des Epiglottishebers 3 verstanden. Durch einen im Vergleich zu allen anderen Videolaryngoskopen sehr geringen Abstand zwischen Spatelspitze 37 und optischer Erfassungseinrichtung 4 kann die Optik direkt vor den Larynxeingang positioniert werden. Von dieser Position aus kann im Gegensatz zum aktuellen Stand der Technik die gesamte Glottis eingesehen werden, da man so an Aryknorpeln, der Epiglottis und am Tubus vorbeischauen kann. Anstatt wie bei der Standardlaryngoskopie die Zunge aufzuladen und nach oben zu verdrängen, um Platz für die Laryngoskopie und die Intubation zu schaffen, wird diese vergleichsweise kleine Spatelspitze 37 um die Zunge herum geführt.

Für eine einwandfreie Funktionsfähigkeit des gesamten Systems ist neben der Länge auch der Krümmungswinkel der Spatelspitze 37 von großer Bedeutung: Die Spatelspitze 37 kann vorteilhaft um ca. 10° nach oben gekrümmt sein, d.h. um ca. 10° relativ zur Unterseite der Führungsschiene 2 in diesem Bereich nach oben abragen. Dadurch werden folgende zwei Funktionen gewährleistet: 1. Das Hochziehen des Epiglottishebers 3 führt selbst bei großen Patienten Ober 180 cm tatsächlich auch zu einem zuverlässigen und effektivem Aufrichten der Epiglottis. 2. Der Krümmungswinkel und die entstehende Öffnung zwischen Spatelspitze 37 und dem freien Ende 24 der Führungsschiene 2 sind noch klein genug, um im unbetätigten Zustand des Epiglottishebers 3 die beiden Spitzen 24, 37 um die Zunge herumführen zu können, Je größer der Winkel und die entstehende, zangenförmige Öffnung ist, um so größer ist die Gefahr, dass die Enden 24, 37 nicht um die Zunge herum, sondern in den Zungenboden anstatt weiter bis zum Larynx geführt werden. Dann kann auch durch Hochziehen des Epiglottishebers 3 keine Sicht auf die Glottis hergestellt werden, da Zungengewebe die Sicht weiter blockiert.

Wie die Figur 2 zeigt, ragt die Spatelspitze 37 etwas, z.B. ca. 1 cm, über das freie Ende 24 der Führungsschiene 2 hinaus und weist einen Krümmungswinkel von 10° relativ zur Unterseite der Führungsschiene 2 auf. Je kleiner Länge und Krümmungswinkel der Spatelspitze 37 sind, desto leichter lassen sich die besser adaptierten Enden 24, 37 von Führungsschiene 2 und Epiglottisheber 3 um die Zunge herumführen.

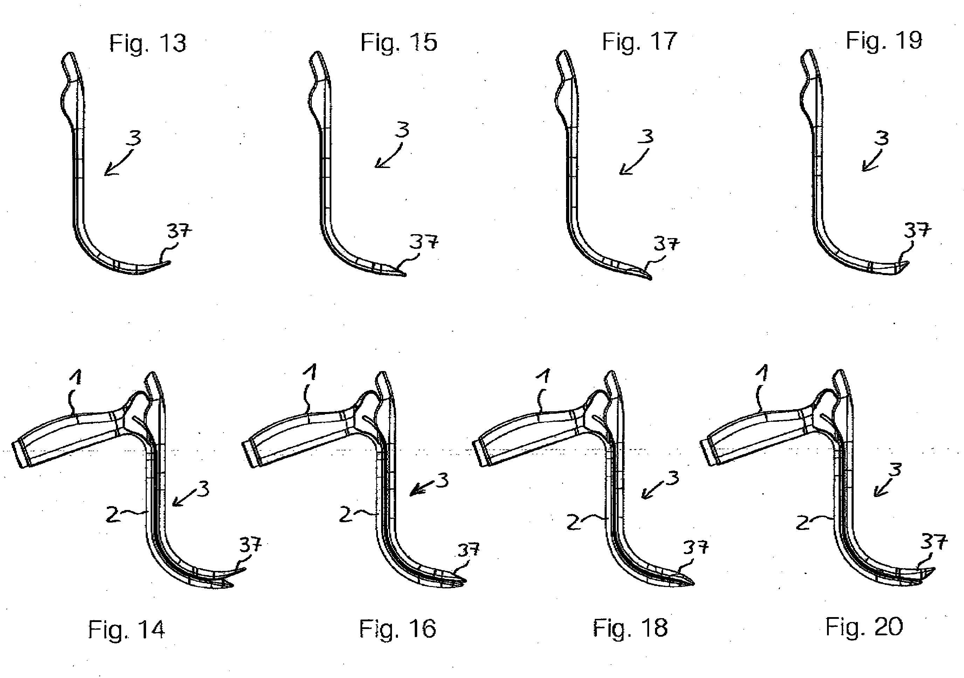

Die Figuren 13 bis 20 verdeutlichen Varianten des Epiglottishebers 3. Dabei ist in den Figuren 13,15,17, und 19 jeweils nur der Epiglottisheber 3 dargestellt, in der jeweiligen darunter abgebildeten Figur 14,16,18 bzw. 20 das gesamte Intubationsgerät mit dem darüber abgebildeten Epiglottisheber 3. Die Spatelspitzen 37 der Varianten in den Figuren 15 bis 18 ragen nicht oder nur sehr wenig über die Führungsschiene 2 hinaus und sie sind auch nicht wie die Standardvariante in den Figuren 13 und 14 nach oben gekrümmt, sondern nach unten zur Führungsschiene 2 hin. Dadurch sind in den Figuren 15 bis 18 die Enden von Führungsschiene 2 und Epiglottisheber 3 wie Ober- und Unterkiefer bei einem Delphin besser adaptiert und die Öffnung geschlossen. Das erleichtert die Passage um die Zunge und insbesondere um den Zungenboden herum.

Allerdings wird bei dieser geraden, abgeflachten Form der Spatelspitze 37 die Epiglottis in geringerem Ausmaß und vor allem nicht so zuverlässig angehoben. Bei zunehmender Körpergröße ist das noch deutlicher der Fall. Das kann die Sicht auf die Glottis und somit auch die Intubation erschweren.

### Querdurchmesser und andere Hebemechanismen

Durch die Form der hervorstehenden Spatelspitze 37 wird auch in diesem Bereich ein geringer Gesamtdurchmesser erreicht und somit das Einführen auch bei geringer Mundöffnung gewährleistet. Wie bereits oben dargestellt, bleibt durch das Funktionsprinzip des Epiglottishebers 3 auch nach Öffnung des Atemwegs der Querdurchmesser auf Mundhöhe und im oberen Rachenbereich unter 2 cm.

Wenn der Epiglottisheber 3 nicht verschoben wird, wie in Figur 3 dargestellt, sondern wie in Figur 4 dargestellt gekippt oder herunter gedrückt wird, wird der Durchmesser nicht nur im unteren, sondern auch im oberen Rachenbereich erhöht. Dadurch ist ein erhöhter Kraftaufwand erforderlich und die Öffnung kann nicht so effektiv erfolgen.

Hier sind andere Mechanismen vorteilhaft, die die Zunahme des Querdurchmessers im oberen Rachenbereich minimieren oder verhindern. Außer der oben beschriebenen Methode des Hochziehens kann das auch durch Versetzen des Drehpunktes weiter nach unten erreicht werden. Außerdem kann der untere Bereich auch mit einen Kippspatel eröffnet werden, ohne den Querdurchmesser im oberen Bereich weiter eröffnen zu müssen.

### Schutz der optischen Erfassungseinrichtung 4 - "Dach" und Seitenränder

Oben wurde beschrieben, welche wichtigen Funktionen durch die herausragende Spatelspitze 37 und den Krümmungswinkel gewährleistet werden. Diese Form der Spatelspitze 37 bewirkt auch, dass Sekret und Zungengewebe auf Abstand gehalten und Sichtblockaden der optische Erfassungseinrichtung 4 in der Führungsschiene 2 in den meisten Fällen verhindert werden können.

Darüber hinaus wird der Schutz der Optik in der Führungsschiene 2 durch seitlich abgerundete Ränder und vor allem durch ein "Dach" im Bereich 30 des Epiglottishebers 3 gewährleistet. Der Epiglottisheber 3 kann von der Spatelspitze 37 bis zum unteren Drittel geschlossen ausgebildet sein. Dadurch wird verhindert, dass weiches Zungengewebe insbesondere in Narkose oder im Koma auf die Optiken gelangen und die Sicht teilweise oder vollständig blockieren kann. Auch ohne Hochziehen gewährleisten Krümmung und Form der Spatelspitze 37 eine freie Sicht und auch einen freien Durchtritt eines Endotrachealtubus mit einem Aussendurchmesser von bis zu 10 mm.

### Stufenloses Anheben des Epiglottishebers3

Wie oben beschrieben erfolgt das Öffnen des tiefen Rachenbereichs und das Anheben der Epiglottis mit dem Epiglottisheber 3 stufenlos und bedarfsgerecht unter Sichtkontrolle. Hierfür kann durch Änderung der Druckausübung beispielsweise mit der Zeigefingerspitze beim nach oben Ziehen mit der anderen Hand jederzeit die gewünschte Position des Epiglottishebers 3 durch Andrücken an die Führungsschiene 2 fixiert und gehalten werden. Diese Funktion kann auch von einem Befestigungsring oder einer sonstigen ersten Fixiervorrichtung 10 übernommen werden.

### Richtungswechsel bei Bewegungen des Epiglottishebers 3

Bei Bedarf kann der Epiglottisheber 3 nicht nur nach oben, sondern auch wieder nach unten bewegt und somit die Position unter Sichtkontrolle korrigiert und wie - gewünscht angepasst werden. Solche Richtungskorrekturen sind besonders dann erforderlich, wenn der Epiglottisheber 3 wieder in die Ausgangsposition zurückbewegt werden soll. Das ist die Voraussetzung, um ihn zusammen mit der Führungsschiene 2 in geschlossenem Zustand wieder aus dem Mund herausziehen zu können. Das wird dann nötig, wenn eine Intubation mit einem anderen Tubus erfolgen soll oder keine Intubation, sondern eine andere Intervention erfolgt. Das ist zum Beispiel die Applikation von Lokalanästhesie auf den Larynxeingang. Dann ist das Herausziehen des ganzen, geschlossenen Intubationsgeräts einfacher, als Epiglottisheber 3 und Führungsschiene 2 zu trennen und einzeln herauszunehmen, wie es nach einer Intubation erforderlich ist.

### Verankerung, Tubusführung und Trennung von Tubus, Epiglottisheber und Führungsschiene

Nach der Intubation muss beim Entfernen des Intubationsgeräts die akzidentelle Extubation mit an Sicherheit grenzender Wahrscheinlichkeit ausgeschlossen sein. Hierfür wird der Epiglottisheber 3 sehr einfach und schnell von der Führungsschiene 2 getrennt: Dazu wird der Epiglottisheber 3 noch weiter nach oben gezogen, wobei er sich von seiner durch die formschlüssigen Befestigungselemente 25, 35 bewirkten Fixierung mit der Führungsschiene 2 löst. Er wird dann nach vorne geführt, um ihn aus dem Mund zu ziehen.

Bei der Fixierung handelt es sich z.B. um eine ca. 10 mm lange Vorwölbung am Epiglottisheber 3, die hinter eine Vorwölbung am oberen Rand der Führungsschiene 2 geführt wird. Hierdurch entsteht eine lose Verbindung, die aber ausreicht, um ein versehentliches Ablösen des Epiglottishebers 3 von der Führungsschiene 2 zu verhindern. Erst wenn die Vorwölbung des Epiglottishebers 3 über den entsprechenden Rand der Führungsschiene 2 gezogen wird, löst sich die Verankerung zwischen Epiglottisheber 3 und Führungsschiene 2. Durch diesen einfachen Mechanismus muss der Epiglottisheber 3 nicht aktiv an der Führungsschiene 2 festgehalten werden. Diese Verankerung verhindert, dass der Epiglottisheber 3 sich,versehentlich wieder von der Führungsschiene 2 löst - insbesondere, wenn das Intubationsgerät nach vorne geneigt wird. Diese einfache Fixierung gewährleistet andererseits, dass wie oben beschrieben durch einfaches nach oben Ziehen die Verbindung zwischen Epiglottisheber 3 und Führungsschiene 2 schnell und unkompliziert wieder gelöst werden kann.

Gleichzeitig wird darüber hinaus bei der Lösung der Verankerung durch Ziehen nach oben und Kippen nach vorn der Epiglottisheber 3 auch einfach, schnell und unkompliziert vom Endotrachealtubus getrennt. Hierdurch wird ein versehentliches Entfernen des Endotrachealtubus sehr unwahrscheinlich. Vorteilhaft hierfür ist die vom unteren Drittel des Epiglottishebers 3 bis zum Bedienelement 32 reichende Aussparung 33: Durch diese Öffnung wird vor der Intubation der Endotrachealtubus in den Führungskanal 23 eingeführt und zwischen Führungsschiene 2 und Epiglottisheber 3 eingespannt. Bei der Intubation gewährleistet diese Aussparung 33, das der Endotrachealtubus in seinem mittleren Bereich gegriffen und auch bei problematischen Intubationen mit verschiedenen Manövern wie z.B. Drehbewegungen in die Luftröhre vorgeschoben werden kann.

Nach der Intubation kommt dann der z.B. wenigstens 30 mm grossen Bedienaussparung 34 des ringförmigen Bedienelemnts 32 am oberen Ende des Epiglottishebers 3 eine besondere Bedeutung zu: Diese Öffnung gewährleistet, dass nach der intubation der breite Beatmungskonnektor des Endotrachealtubus leicht hindurchgeführt und somit der Endotrachealtubus vom Epiglottisheber 3 getrennt werden kann. Hierbei wird das Risiko einer versehentlichen Extubation minimiert.

Trennung von Epiglottisheber 3 und Endotrachealtubus: Der Epiglottisheber 3 wird nach vorne gekippt, d.h. im patientenfernen Bereich von der Führungsschiene 2 weg, wobei der Endotrachealtubus durch die Bedienaussparung 34 geführt und so vom Epiglottisheber 3 getrennt wird. Nach der Entfernung des Epiglottishebers 3 wird mit dem Handgriff 1 die Führungsschiene 2 aus dem Mund gezogen, wobei der Endotrachealtubus mit der anderen Hand in Position gehalten wird.

### Übersichtsoptik im Epiglottisheber und Befestigungsmechanismus für Optiken

Die Integration einer Optik, d.h einer optischen Erfassungseinrichtung, im Epiglottisheber 3 stellt eine Zusatzoption für eine unterstützende Übersicht dar. Solch eine ergänzende Übersicht kann zusätzliche zu einem oder zwei in der Führungsschiene 2 befindlichen Optiken in bestimmten Situationen sinnvoll sein: So kann, um sich beispielsweise bei veränderter Anatomie im Rahmen einer malignen Erkrankung besser orientieren zu können, eine zusätzliche Übersichtsperspektive von weiter hinten und oben vorteilhaft sein. Außerdem kann bei problematischen Sichtverhältnissen von dieser Perspektive aus die Position der beiden unteren Optiken in der Führungsschiene 2 relativ zu den anatomischen Strukturen bei solchen schwierigen Verhältnissen besser erfasst und gegebenenfalls korrigiert werden. Das kann nicht nur bei veränderten anatomischen Verhältnissen, sondern auch für Anfänger von Vorteil sein.

So eine Position der Optik ist aber insbesondere dann von Vorteil, wenn sich viel Flüssigkeit im tieferen Rachenbereich ansammelt und nicht effektiv oder schnell genug abgesaugt werden kann. Dies kann zum Beispiel bei Erbrechen oder bei einer ausgeprägten Blutung der Fall sein. Hierbei kann selbst bei einer am tiefsten Punkt unter der Führungsschiene befindlichen, leistungsstarken Absaugung mit Spülsystem eine Sichtblockade der Optiken in der Führungsschiene auftreten. In dieser speziellen Notfallsituation kann dann eine weiter oben gelegene Optik im Epiglottisheber noch eine ausreichende Sicht gewährleisten.

Eine Optik kann fest in den Epiglottisheber 3 eingebaut sein. Es kann aber auch eine Optik wie beispielsweise ein Einweg-Endoskop an der Unterseite des Epiglottishebers 3 befestigt werden: Hierfür können runde Klammern und/oder ein Kanal im Epiglottisheber 3 eingebettet sein. Ein Endoskop kann dann so befestigt werden, dass es rotationsstabil an einer bestimmten Stelle an der Unterseite fixiert wird. Das kann z.B. auf Höhe der Optiken in der Führungsschiene 2 sein. Weitere Befestigungsmöglichkeiten am Epiglottisheber gewährleisten die Rotationsstabilität des Endoskops.

Ein in der Unterseite des Epiglottishebers 3 eingebetteter Befestigungsmechanismus zum Befestigen einer Optik ragt so wenig wie möglich nach unten in den Führungskanal 23 hinein. Hierdurch soll ein ungehindertes Gleiten des Endotrachealtubus auf der Führungsschiene 2 gewährleistet werden. Auch soll eine Beschädigung des Cuffs, wie sie beispielsweise häufig durch die Führungsklammern beim oropharyngealen Atemweg nach Ovassapian vorkommt, vermieden werden.

### Fixierung des Epiglottishebers in der betätigten Stellung und individuell angepasster, oropharyngealer Atemweg

Eine leicht anzubringende und auch wieder zu entfernende erste Fixiervorrichtung 10 fixiert Epiglottisheber 3 und Führungsschiene 2 im oberen Anteil, d.h. im patientenfernen Bereich 9, der beim Intubationsvorgang außerhalb des Patienten ist. Dieser Fixierungsmechanismus drückt Epiglottisheber 3 und Führungsschiene 2 genau so zusammen, dass der Epiglottisheber 3 einerseits frei und uneingeschränkt weiter nach oben und unten bewegt werden kann. Andererseits reicht der Druck aus, um die unter Sichtkontrolle eingestellte Position automatisch zu fixieren. Ein zusätzlicher Druck oder die Aktivierung eines weiteren Mechanismus zur Fixierung ist nicht erforderlich.

Die erste Fixiervorrichtung 10 kann im Epiglottisheber 3 integriert sein oder beispielsweise als separater Befestigungsring um Epiglottisheber 3 und Führungsschiene 2 geführt und dort befestigt werden. Die angebrachte erste Fixiervorrichtung 10 kann im geschlossenem Zustand bedarfsweise nach oben geführt werden, um insbesondere bei sehr großen Patienten die Führungsschiene 2 tiefer einführen zu können. Die angebrachte erste Fixiervorrichtung 10 kann auch nach unten geführt werden, um durch einen Gegendruck bei Kontakt mit Mund oder Zähnen das Intubationsgerät noch stabiler in verschiedenen Ebenen mit einer optimierten Glottissicht fixieren zu können.

Sobald die Glottissicht eingestellt wird, bildet das Intubationsgerät hierdurch einen sicheren, individuell angepassten oropharyngealen Atemweg. Er hält den Atemweg zuverlässig offen, da bei der Einstellung der Glottissicht die optimale Position für einen oropharyngealen Atemweg erreicht wurde: Die an der Rachenhinterwand anliegende Epiglottis, Zungengewebe und andere Atemwegsstrukturen wurden effektiv angehoben bzw. eröffnet. Das ermöglicht nicht nur eine sichere Intubation. Dieser individuell angepasste und daher optimale, oropharyngeale Atemweg hält die oberen Atemwege sicher und zuverlässig offen und gewährleistet somit eine ungehinderte Atmung.

Darüber hinaus kann jetzt unter Sichtkontrolle auch eine kontinuierliche Absaugung von Flüssigkeit am tiefsten Punkt des Rachens erfolgen, z.B. mit einem Saug-SpülSystem. Auch andere Maßnahmen können jetzt an den zuverlässig geöffneten Atemwegen durchgeführt werden.

### Selbstständige Fixierung der Laryngoskopieposition im Patienten

Durch die oben beschriebene Fixierung in einer geöffneten Stellung mittels der ersten Fixiervorrichtung 10 muss das Intubationsgerät nicht mehr festgehalten oder anderweitig beispielsweise über eine Stütze oder eine andere Halterung fixiert werden, um Interventionen am Larynx vornehmen zu können. Die Fixierung des Epiglottishebers 3 an der Führungsschiene 2 in der betätigten Stellung fixiert also zusätzlich selbstständig beim liegenden Patienten das gesamte Intubationsgerät in der gewünschten Position mit der erreichten Einstellung für eine optimierte Sicht auf den Larynxeingang: Das Eigengewicht von Zunge und Halsweichteilen drückt die Führungsschiene 2 nach unten an die Rachenhinterwand. Hierdurch wird die Position mit der eingestellten Glottissicht selbständig und sicher gehalten. Ein Festhalten mit der Hand oder eine anderweitige Fixierung ist wie bereits erwähnt nicht mehr erforderlich.

Durch die fixierte, sichere Eröffnung der Atemwege werden beide Hände von der Bedienung des Intubationsgerätes freigesetzt. Es können nun also auch interventionen bzw. Maßnahmen ohne weitere Assistenz durchgeführt werden: So kann beispielsweise sicher ein flexibles Endoskop in die Trachea geführt werden, ohne dass jemand die Atemwege offen halten muss. Das geschieht sogar unter anhaltender Glottissicht selbst nach Einführen des Endoskops in die Trachea. Außerdem gibt es speziell gebogene chirurgische Instrumente, um entlang der natürlichen Krümmung der Atemwege Eingriffe am Larynx vornehmen zu können. Mit der im Patienten fixierten Laryngoskoposition lassen sich diese Instrumente nun einfach zum Larynx führen. Wegen der sehr viel geringeren Belastung durch diese viel schonendere Form sowohl der Öffnung der Atemwege als auch dieser Fixierungweise können so bisher in Narkose vorgenommene Eingriffe jetzt in viel weniger belastender, örtlicher Betäubung erfolgen.

### Bezugszeichenliste

- 1: Handgriff
- 2: Führungsschiene
- 3: Epiglottisheber
- 4: Optische Erfassungseinrichtung
- 5: Erfassungsrichtung der optischen Erfassungseinrichtung
- 6: Oberseite der Führungsschiene
- 7: Unterseite der Führungsschiene
- 8: Patientennaher Bereich
- 9: Patientenferner Bereich
- 10: Erste Fixiervorrichtung

- 20: Biegeabschnitt der Führungsschiene
- 21: Gerader Bereich der Führungsschiene
- 22: Auflagerungflächen der Führungsschiene
- 23: Führungskanal
- 24: Freies Ende (Spitze) der Führungsschiene 2 ,
- 25: Zweites formschlüssiges Befestigungselement

- 30: überwiegend oder vollständig geschlossener Abschnitt
- 31: Seitensteg
- 32: Bedienenelement
- 33: Schlitzförmige Aussparung
- 34: Bedienaussparung
- 35: Erstes formschlüssiges Befestigungselement
- 36: Auflagerungflächen des Epiglottishebers
- 37: Spatelspitze
- 38: Verjüngung

- R: Krümmungsradius der Führungsschiene 2 in Seitenansicht
- Z: Zugkraft am Bedienelement 32

## Patentansprüche

1. Intubationsgerät zum Intubieren eines Patienten mit einem Endotrachealtubus, mit folgenden Merkmalen:
a) einem Handgriff (1) zum Halten des Intubationsgeräts,
b) eine mit dem Handgriff (1) verbundene Führungsschiene (2), die einen in Längsrichtung von einem patientenfemen Ende (9) bis zu einem patientennahen Ende (8) der Führungsschiene (2) verlaufenden Führungskanal (23) zum Führen des Endotrachealtubus und eine beim Intubationsvorgang der Zunge des Patienten zugewandte Oberseite (6) hat,
c) einem Epiglottisheber (3) zum Anheben der Epiglottis des Patienten, wobei der Epiglottisheber (3) über wenigstens ein Lagerungselement an einem Bauteil des Intubationsgeräts beweglich gelagert ist,
d) wenigstens eine optische Erfassungseinrichtung (4) im patientennahen Bereich (8) an der Führungsschiene (2) und/oder wenigstens eine optische Erfassungseinrichtung (4) im patientennahen Bereich (8) an dem Epiglottisheber (3),
e) wobei der Epiglottisheber (3) zumindest im patientennahen Bereich (8), insbesondere in einem Biegeabschnitt (20) der Führungsschiene (2), die Führungsschiene (2) oder zumindest den Führungskanal (23) auf der Oberseite (6) der Führungsschiene (2) überwiegend oder vollständig geschlossen überdeckt.

2. Intubationsgerät nach Anspruch 1, **dadurch gekennzeichnet, dass** der Epiglottisheber (3) am patientenfernen Ende des überwiegend oder vollständig geschlossenen Bereichs (30) in einen offenen Bereich übergeht, der eine schlitzförmige Aussparung (33) hat, der seitlich jeweils von einem Seitensteg (31) begrenzt ist.

3. Intubationsgerät nach einem der vorhergehenden Ansprüche, **dadurch gekennzeichnet, dass** der Epiglottisheber (3) in dem überwiegend oder vollständig geschlossenen Bereich (30) entgegengesetzt U-förmig zur Führungsschiene (2) ausgebildet ist.

4. Intubationsgerät nach einem der vorhergehenden Ansprüche, **dadurch gekennzeichnet, dass** der Epiglottisheber (3) zumindest in dem überwiegend oder vollständig geschlossenen Bereich (30) die Führungsschiene (2) beidseitig übergreift.

5. Intubationsgerät nach einem der vorhergehenden Ansprüche, **dadurch gekennzeichnet, dass** der Epiglottisheber (3) über das wenigstens eine Lagerungselement relativ zum Handgriff (1) bewegbar ist, so dass durch eine Relativbewegung des Epiglottishebers (3) gegenüber dem Handgriff (1) die Epiglottis beim Intubationsvorgang anhebbar ist.

6. Intubationsgerät Intubationsgerät nach einem der vorhergehenden Ansprüche, **dadurch gekennzeichnet, dass** der Epiglottisheber (3) über das wenigstens eine Lagerungselement an der Führungsschiene (2) gleitend verschiebbar und/oder schwenkbar gelagert ist.

7. Intubationsgerät nach einem der vorhergehenden Ansprüche, **dadurch gekennzeichnet, dass** der Epiglottisheber (3) stufenlos oder feinstufig in einer Vielzahl von Stufen relativ zum Handgriff (1) bewegbar ist.

8. Intubationsgerät nach einem der vorhergehenden Ansprüche, **dadurch gekennzeichnet, dass** das Intubationsgerät eine erste Fixiervorrichtung (10) hat, mit der eine eingestellte Relativposition des Epiglottishebers (3) stufenlos oder feinstufig in einer Vielzahl von Stufen fixierbar ist.

9. Intubationsgerät nach einem der vorhergehenden Ansprüche, **dadurch gekennzeichnet, dass** der Epiglottisheber (3) wenigstens ein erstes formschlüssiges Befestigungselement (35) hat, das zumindest im unbetätigten Zustand des Epiglottishebers (3) im formschiüssigen Eingriff (1) mit einem zweiten formschlüssigen Befestigungselement (25) des Intubationsgeräts ist.

10. Intubationsgerät nach einem der vorhergehenden Ansprüche, **dadurch gekennzeichnet, dass** die Führungsschiene (2) zumindest in einem patientennahen Bereich (8) einen Biegeabschnitt (20) aufweist, in dem die Führungsschiene (2) von der Seite aus betrachtet, an der der Handgriff (1) angeordnet ist, konvex gebogen ist.

11. Intubationsgerät nach Anspruch 10, **dadurch gekennzeichnet, dass** der Führungskanal (23) auf der Innenseite des Biegeabschnitts (20) überwiegend oder vollständig offen ist.

12. Intubationsgerät nach einem der Ansprüche 10 bis 11, **dadurch gekennzeichnet, dass** die Führungsschiene (2) am patientenfernen Ende des Biegeabschnitts (20) in einen linear verlaufenden oder im Vergleich zum Biegeabschnitt weniger stark gekrümmten Abschnitt (21) übergeht.

13. Intubationsgerät nach einem der vorhergehenden Ansprüche, **dadurch gekennzeichnet, dass** das Intubationsgerät eine zweite Fixiervorrichtung hat, mit der die Lage des Intubationsgeräts relativ zum Patienten, insbesondere die Winkellage, stufenlos oder feinstufig in einer Vielzahl von Stufen fixierbar ist.
